# EUROPEAN PATENT SPECIFICATION

(11) **EP 1 905 455 B1**
(45) Date of publication and mention of the grant of the patent: **24.07.2013**
(21) Application number: 06747284.5
(22) Date of filing: 06.06.2006
(51) Int. Cl.: A61K 47/36, A61K 47/48, A61K 47/04, A61K 47/10, A61K 47/14, A61K 47/42, A61K 47/44, A23L 1/00, A61K 9/48

(54) **CAPSULE COATING COMPOSITION**
KAPSELHALTIGE ZUSAMMENSETZUNG
COMPOSITION DE REVETEMENT DE CAPSULES

(30) Priority: 06.06.2005 JP 2005165652
(43) Date of publication of application: 02.04.2008
(73) Proprietor: Ajinomoto Co., Inc., Tokyo 104-8315 (JP)
(72) Inventor: SATOKAWA, Hideo, c/o AJINOMOTO CO., INC., Kawasaki-shi, Kanagawa 2108681 (JP); FURUTA, Kiyonori, c/o AJINOMOTO CO., INC., Kawasaki-shi, Kanagawa 2108681 (JP); IWASAKI, Keiji, c/o AJINOMOTO CO., INC., Kawasaki-shi, Kanagawa 2108681 (JP)
(74) Representative: Nicholls, Kathryn Margaret
(86) International application number: PCT/JP2006/311695
(87) International publication number: WO 2006/132398

(56) References cited:
- WO-A1-98/58654
- WO-A1-03/049771
- JP-A- 03 047 087
- JP-A- 05 097 694
- JP-A- 58 085 813
- JP-A- 2000 139 372
- JP-A- 2000 336 028
- JP-A- 2003 055 198
- JP-A- 2003 221 323
- JP-A- 2003 504 326
- JP-A- 2004 534 023
- US-A- 4 803 168
- DATABASE WPI Week 200533 Thomson Scientific, London, GB; AN 2005-316186 XP002669557, & CN 1 561 971 A (HUBEI ENG COLLEGE) 12 January 2005 (2005-01-12)

## Description

### Technical Field

The present invention relates to a composition for producing a capsule shell, which comprises (A) a vegetable polysaccharide and (B) polyglutamic acid (sometimes to be referred to as a composition for a capsule shell), and further relates to a composition for a capsule shell comprising (A) vegetable polysaccharide, (B) polyglutamic acid, and at least one kind selected from C) water, (D) a plasticizer, (E) glycerin fatty acid ester and (F) oil, a capsule shell sheet and a capsule preparation.

### Background Art

A capsule shell is generally produced using gelatin as a main component. However, gelatin is not a preferable material in recent years for religious people and vegetarians because it is derived from animal, and moreover, from the fear of mad cow disease.

In addition, production of a soft capsule using gelatin as a main component requires heating for a long time from dissolution to during production. Also, production of a soft capsule of a constant quality is problematically difficult because the heating changes the structure of gelatin, decreasing the viscosity of liquid and the like.

From such background, some capsules using a plant-derived one have been developed.

JP-A-2003-504326 discloses a soft capsule using I-carageenan and modified starch as vegetable starting materials. However, the use of starch and I-carageenan in combination as starting materials requires a large amount of water for dissolving the starting materials. Therefore, drying of the capsule becomes a heavy burden, and the productivity is not entirely good.

JP-A-2003-55198 discloses a capsule based on a combination of water-soluble etherified starch as a vegetable starting material and a plasticizer. However, since adhesiveness of capsule shell sheets during capsule formation is insufficient, sufficient strength cannot be entirely achieved during capsule formation.

JP-A-2003-221323 discloses a soft capsule shell sheet containing a decomposition product of hydroxypropyletherified starch. However, since sufficient adhesiveness cannot be achieved even when combined with a plasticizer such as sorbitol and the like and the capsule shell sheet lacks flexibility, forming is not necessarily easy.

WO 03/049771 discloses a capsule shell sheet containing polyglutamic acid (PGA). However, preparation of a capsule containing PGA requires first drying a capsule shell sheet formed from a shell solution containing PGA over a long time. Accordingly, problems occur in that capsules cannot be produced continuously, and caking occurs when a desiccant is not used during preservation due to the hygroscopicity of the capsule, and the sheet is not necessarily sufficient.

WO 02/089764 describes a capsule containing a plant-derived water-soluble protein, and further teaches that a gelling agent such as carrageenan and a plasticizer are contained.

The problem to be solved by the present invention is to provide a composition for a shell, which is suitable for producing a plant-derived, practical capsule shell sheet having both sufficient strength and elongation as a capsule shell, superior adhesiveness and transparency, and a low burden on a drying step, a capsule shell sheet obtained using the composition for a shell, and further a capsule preparation.

### Disclosure of the Invention

The present inventors have conducted intensive studies in an attempt to solve the above-mentioned problems and found that the objective composition for a capsule shell can be obtained by mixing (A) vegetable polysaccharide, (B) polyglutamic acid, and (C) water and, where necessary, at least one kind selected from (D) a plasticizer, (E) glycerin fatty acid ester and (F) oil.

That is, the present invention includes the following embodiments.
[1] A composition for a capsule shell, which comprises (A) a vegetable polysaccharide and (B) polyglutamic acid.
[2] The composition of the above-mentioned [1], which further comprises (C) water.
[3] The composition of the above-mentioned [1] or [2], which further comprises (D) a plasticizer.
[4] The composition of any of the above-mentioned [1] to [3], which further comprises (E) a glycerin fatty acid ester.
[5] The composition of any of the above-mentioned [1] to [4], which further comprises (F) an oil.
[6] The composition of any of the above-mentioned [1] to [5], wherein (A) is a starch.
[7] The composition of the above-mentioned [6], wherein the starch is selected from tapioca starch, corn starch, waxy corn starch, rice starch and wheat starch.
[8] The composition of any of the above-mentioned [1] to [7], wherein (A) is a modified vegetable polysaccharide.
[9] A capsule shell sheet, which comprises (A) a vegetable polysaccharide and (B) polyglutamic acid.
[10] A capsule preparation comprising (A) a vegetable polysaccharide and (B) polyglutamic acid.

### Best Mode for Embodying the Invention

The embodiment of the present invention is explained in detail in the following by referring to the best mode.

The composition for a capsule shell of the present invention contains at least vegetable polysaccharides and polyglutamic acid.

The kind of (A) vegetable polysaccharides to be used for the composition for a capsule shell of the present invention is not particularly limited as long as it is a general starting material derived from a plant, and may be a natural product, a processed product and the like. Specifically, starch such as tapioca starch, corn starch, waxy corn starch, high-amylose corn starch, maize starch, potato starch, rice starch, wheat starch and the like, carageenan, agar, gellan gum, hydroxypropylmethylcellulose, sodium alginate and the like can be used. From the aspect of elongation and elasticity suitable for a capsule shell sheet, starch is preferable, and tapioca starch, corn starch, waxy corn starch, rice starch and wheat starch are more preferable.

When a starch is selected as a main component of (A), a property to prevent sticking of capsules during a drying step can be imparted by combining a component other than the starch. While the component other than the starch is not particularly limited, carageenan, gellan gum, glucomannan and agar are preferable, carageenan and gellan gum are more preferable, and carageenan is particularly preferable. The component to be blended besides starch shows effect even at an amount of about 0.1 - 5% of the total amount of component A. When the amount is less than 0.1%, a clear sticking-preventive effect may not be achieved sometimes, and when it exceeds 5%, the transparency of the sheet may be influenced. To provide,a useful effect, the amount is more preferably 0.2 - 3%, particularly preferably 0.5 - 2%.

Generally, (A) vegetable polysaccharides to be used for the composition for a capsule shell of the present invention may or may not be modified. As a specific modification method, a method based on hydroxypropyletherification, carboxymethylation and the like can be employed. From the aspect of elongation and hardness suitable for a capsule shell sheet, vegetable polysaccharides are preferable, vegetable polysaccharides modified by hydroxypropylation are more preferable, and modified vegetable polysaccharides decomposed by enzyme are more preferable.

When a modified vegetable polysaccharide is used as (A) vegetable polysaccharide to be used for the composition for a capsule shell of the present invention, one free of decomposition may be generally used or a decomposed one may be used. As a specific method of decomposition, decomposition by heat, enzyme, acid and the like can be used. From the aspect of improvement in the elongation and elasticity suitable for a capsule shell sheet, a modified vegetable polysaccharide decomposed by enzyme is preferable.

While (B) polyglutamic acid to be used for the composition for a capsule shell of the present invention is not particularly limited, for example, synthetic poly-α-glutamic acid, poly-γ-glutamic acid present in a viscous substance of *natto* or extrabacterially secreted by the *Bacillus* such as *Bacillus natto* and the like, a salt thereof and the like can be used. Since these (B) polyglutamic acids can improve water solubility of vegetable polysaccharides, they can decrease the amount of water in the composition for a capsule shell. In addition, they improve adhesiveness of the capsule shell sheet as well as show a mineral solubilization effect in the lower section of the small intestine, thus promoting intestinal absorption of minerals. When used as a food material, poly-α-glutamic acid may be decomposed by protease during passage in the intestine, depending on the conditions of amount and the like. From the aspect of affording a high promoting effect on mineral absorption by the intestine, therefore, poly-γ-glutamic acid present in a viscous substance of *natto* or extrabacterially secreted by the *Bacillus* such as *Bacillus natto* and the like is preferable.

While the molecular weight of (B) polyglutamic acid to be used for the composition for a capsule shell of the present invention is not particularly limited, one having a molecular weight of 3,000 - 1,000,000 is generally used. When it is less than 3,000, the adhesiveness improving effect on the capsule shell sheet is insufficient, and when it exceeds 1,000,000, dissolution and the like in a processing step become difficult. To maintain stable adhesion and the dissolution property of the starting materials, it is preferably 10,000 - 100,000. The molecular weight is defined by a weight average molecular weight obtained using a gel permeation chromatography coupled to multi-angle laser light scattering method (GPC-MALLS method: Dawn DPS manufactured by Wyatt Technology).

The (B) polyglutamic acid to be used for the composition for a capsule shell of the present invention may be in a free form or may form a salt. The salt is not particularly limited and, for example, alkali metals such as sodium, potassium and the like, alkaline earth metals such as magnesium, calcium etc., and the like are used. From the aspect of versatility, sodium salt is preferable. As the kind of the salt, one kind may be used, or two or more kinds may be used in a mixture.

The amounts of (A) and (B) to be used for the composition for a capsule shell of the present invention can be appropriately determined depending on the kind of capsule and the kind of vegetable polysaccharides to be used, from the range where the capsule form and strength can be maintained. As the mixing ratio (mass ratio) of (A) and (B), the range of 99:1 - 30:70 is preferable. Particularly, in the case of a capsule using a starch shell, the mixing ratio (mass ratio) of (A) and (B) is within the range of 99:1 - 60:40. When (A) exceeds 99:1, capsule forming becomes difficult because starch is hardly dissolved in water, resulting in an opaque sheet, and two capsule shell sheets are not adhered sufficiently by heat. On the other hand, when (B) exceeds 60:40, the strength of the capsule shell sheet becomes insufficient, and therefore, production of capsule becomes difficult. From the aspect of comprehensively securing the transparency, adhesiveness, strength and elongation necessary for a capsule shell sheet, the mixing ratio of (A) and (B) is preferably 95:5 - 60:40, more preferably 95:5 - 70:30, further preferably 95:5 - 80:20, and particularly preferably 90:10 - 80:20.

The composition for a capsule shell of the present invention contains (C) water. The (C) water to be used is not particularly limited as long as it is generally used for food. The component (C) is used to mainly dissolve component (A) and component (B), and to improve the capsule property. Here, the amount of water means a total amount of water contained in each component and water to be added during production of the composition. Examples of the water to be added include water used for dissolving or suspending each component as necessary, water used for dissolving or suspending the composition obtained by mixing each component, and the like.

The amount of (C) water contained in the composition for a capsule shell of the present invention is generally within the range of 20 - 55%, when calculated as a mass% to the total amount of those used as the capsule shell components from the components (A) to (F). When the amount is less than 20%, the solid contents may not be dissolved, and when it exceeds 55%, practical use may be difficult because of the heavy burden on a drying step. From the aspect of low burden on the capsule drying step, the upper limit of the water content is preferably 47%, more preferably 41%, and most preferably 35%. On the other hand, the lower limit of the water content is preferably 20%, more preferably 25%, and particularly preferably 30%, from the aspect of low burden on a drying step. When a step of high temperature high pressure treatment (dissolution treatment) using an extruder such as a real producing machine, the amount of (C) water to be added may shift toward 5 to 10% lower.

By further adding (D) a plasticizer to the composition for a capsule shell of the present invention, a capsule shell sheet after film forming or a capsule can be softened. While (D) is not particularly limited, specifically, polyvalent alcohols such as glycerol, propylene glycol, 1,3-butyleneglycol and the like, sugar alcohols such as sorbitol, maltitol, erythritol, lactitol, xylitol and the like, saccharides such as glucose, fructose, sucrose and the like can be used. These may be used alone or two or more kinds thereof may be used in a mixture.

The weight of (D) relative to the total weight of (A) and (B) can be within the range of 5 - 40%. When the weight is less than 5%, a capsule shell sheet may show degraded adhesiveness and also decreased transparency, and when the weight exceeds 40%, the sheet becomes too soft, sometimes resulting in poor strength and poor adhesiveness. Since the total evaluation of adhesiveness, transparency, elongation and strength becomes high, the lower limit is preferably 8%, more preferably 10%, and particularly preferably 12%. The upper limit is preferably 30%, more preferably 25%, further preferably 22%, and particularly preferably 18%.

By further adding (E) a glycerin fatty acid ester in an amount within a certain range, the composition for a capsule shell of the present invention can enhance the coat strength of the capsule shell sheet.

The weight of (E) relative to the total weight of (A) and (B) is within the range of 0.1 - 25%. When the weight is less than 0.1%, the strength of the capsule shell sheet may become low, and when it exceeds 25%, the strength of the capsule shell sheet may become low. From the aspect of production of a capsule shell sheet or capsule more superior in the strength, the lower limit is preferably 0.5%, more preferably 1%, further preferably 2%, still more preferably 4%, especially preferably 7%, and particularly preferably 10%. The upper limit is preferably 22%, more preferably 20%, further preferably 17%, and particularly preferably 15%.

By further adding (F) an oil to the composition for capsule shell of the present invention, the detachability of the capsule shell sheet can be improved. (F) is not particularly limited, and soybean oil, canola oil, safflower oil, corn oil, cocoanut oil and palm oil can be specifically mentioned. These may be used alone or two or more kinds thereof may be used in a mixture. To improve detachability, corn oil, cocoanut oil and palm oil are preferable, cocoanut oil and palm oil are more preferable, and palm oil is particularly preferable.

The weight of (F) relative to the total weight of (A) and (B) is within the range of 0.1 - 6%. When the weight is less than 0.1%, the detachability may be poor, and when the weight exceeds 6%, the strength may become low. Since a capsule shell sheet having strength and superior detachability can be obtained, the lower limit is preferably 0.2%, more preferably 0.4%, and particularly preferably 0.5%. The upper limit is preferably 5.0%, more preferably 3.0%, and particularly preferably 1.5%.

The capsule shell sheet of the present invention can contain other components generally used as shell components to the extent that does not inhibit the effect of the present invention. For example, additives known and in public use such as coloring agent, shielding agent, preservative, stabilizer, buffer and the like can be used.

The capsule shell sheet of the present invention can be adapted to any form of various capsules such as soft capsule and seamless soft capsule.

The shape, size and the like of capsule are not particularly limited. As for the form, round type, oval type, oblong type, tube type, teardrop type and the like can be produced. As for the size, a capsule of the size of several micrometers to several centimeters can be produced.

The composition for a shell of the present invention is processed to give a sheet for capsule with an extruder generally used for food processing, and then the capsule shell sheet is subjected to a generally-used soft capsule production machine according to the method described in JP-A-2003-70428 and the like. Specifically, firstly, a composition for a shell is treated at a high temperature and high pressure in an extruder, and the extruded material is formed into a capsule shell sheet with a thickness of about 0.8 mm using a die and a roll. The capsule shell sheet is fed, for example, into a rotary soft capsule production machine to give soft capsules. In the rotary soft capsule production machine, the capsule shell sheet is fed in between a pair of rotary cylindrical molds, and the content of the capsule is injected between the capsule shell sheets with a pump that works along therewith. At this time, the junction parts of the capsule shell sheet are heat-sealed by high pressure cut-off between convex teeth and teeth on the surface of a rotary cylindrical mold while heating to a suitable temperature to give a soft capsule.

Thereafter, the capsule is subjected to a drying step to dry to a desired water content of 10 - 12%, whereby a soft capsule can be obtained. In the case, the drying step is performed using a tumbler dryer and drying in shelf. In the case of a conventional capsule made only of starch, a large amount of water (about 50%) is required because it is not easily dissolved, and the drying time problematically becomes long. However, since the composition for capsule shell of the present invention can suppress the amount of water to be added, the drying time can be shortened and the productivity can be advantageously improved.

The capsule shell sheet of the present invention can also be made to contain the content components simultaneously with the capsule production in advance, whereby a capsule preparation can be formed. The content components may be other components generally used as the content of a capsule preparation. The form of the content components may be any of a liquid, a solid, and a suspension requiring particularly strong adhesiveness can also be encapsulated.

The capsule preparation of the present invention is widely useful in the field of pharmaceutical product, food, health food and the like as a practical capsule derived from a vegetable, which replaces conventional gelatin capsules.

### Examples

The present invention is explained in more detail in the following by referring to Examples, which are not to be construed as limitative.

The evaluation methods in the Examples are explained. (1) Property test of capsule shell sheet

A 1 mm-thick silicone sheet frame was placed on a 10 cm square PET film, each composition was flown in, and a 10 cm square PET film was placed thereon. This was sandwiched with steel plates and fixed with screws, and heated for 5 min in an oil bath heated to 120°C to give a capsule shell sheet. The obtained capsule shell sheet was evaluated for adhesiveness, film strength, elongation, and transparency.
<Adhesiveness>: Two test pieces (1 cmx2 cm) were cut out from the capsule shell sheet obtained in the above, placed on one another and adhered with a heat sealer (Fuji Impulse Co., Ltd. P-200). After adhesion, the two pieces of capsule shell sheet were manually peeled off and the adhesiveness was evaluated. According to the dial setting of the heat sealer, the sheet was judged in 4 levels in Table 1. As used herein, adhesion means that the adhered surfaces of two pieces of sheets are melt fused to lose interface, and two pieces of sheets are not released on peeling.

**Table 1**

| evaluation item | evaluation |
|---|---|
| adhered at dial 6 | ⊙ |
| adhered at dial 7 | ○ |
| adhered at dial 8 | Δ |
| not adhered at all at dials 6 - 8 | × |

(In Table 1, the number of "dial" means heating for the time set. The length of time is dial 6 < dial 7 < dial 8.) <Transparency>: The transparency of the capsule shell sheet was visually evaluated in 4 levels as shown in Table 2.

**Table 2**

| evaluation item | evaluation |
|---|---|
| completely transparent | ⊙ |
| somewhat transparent | ○ |
| opaque | Δ |
| completely opaque | × |

<Intensity and elongation of capsule shell sheet>: Film strength and elongation were evaluated using an all-purpose texture analyzer (SMS, TA.XT plus). The capsule shell sheet obtained above was set to a stage with a hole of diameter 150 mm. A rod with a ball (diameter 130 mm) on the tip was thrust into the surface of a capsule shell sheet at a rate of 0.5 cm/min, and the maximum strength and elongation at that time were measured.

As for the strength, the force applied on breakage was measured. The evaluation levels are as shown in Table 3.

**Table 3**

| evaluation range | evaluation |
|---|---|
| not less than 3.0 MPa | ⊙⊙ |
| not less than 2.8 MPa and less than 3.0 MPa | ○⊙ |
| not less than 2.6 MPa and less than 2.8 MPa | ⊙ |
| not less than 2.4 MPa and less than 2.6 MPa | ○ |
| not less than 2.2 MPa and less than 2.4 MPa | Δ |
| less than 2.2 MPa | × |

As for elongation, the length of elongation on breakage was measured. The evaluation levels are as shown in Table 4.

**Table 4**

| evaluation range | evaluation |
|---|---|
| not less than 10 mm | ⊙⊙ |
| not less than 9 mm and less than 10 mm | ○⊙ |
| not less than 8 mm and less than 9 mm | ⊙ |
| not less than 7 mm and less than 8 mm | ○ |
| not less than 6 mm and less than 7 mm | Δ |
| less than 6 mm | × |

### <Detachability>

In general, the surface of a capsule shell sheet comprising starch as a main starting material has considerably high stickiness as compared to a gelatin capsule shell sheet. Thus, continuous supply to a capsule forming machine is difficult. In addition, capsules produced from a highly sticky capsule shell sheet easily to each other. Therefore, production of a capsule shell sheet affording good detachability of the surface of a capsule shell sheet is important for continuous supply of a capsule shell sheet to a capsule forming machine.

The detachability was evaluated in 4 levels as shown in Table 5, based on the texture of a capsule shell sheet by sandwiching the sheet between fingers and releasing same.

**Table 5**

| evaluation item | evaluation |
|---|---|
| no sticking to finger | ⊙⊙ |
| sticky on fingers but immediately released by loosening hold | ○⊙ |
| capsule shell sheet stretches between fingers and finally released therefrom | ⊙ |
| capsule shell sheet considerably stretches between fingers and finally released therefrom | ○ |
| is not released from finger with ease | Δ |
| is not released from finger at all | × |

### <Evaluation of burden on drying step>

A higher water content requires a longer time for a capsule drying step, and therefore, capsules were evaluated based on the criteria of Table 6.

**Table 6**

| amount of water added (%) | burden on drying step |
|---|---|
| - 35 | ⊙○ (very light) |
| 36 - 41 | ⊙ (light) |
| 42 - 47 | ○ (average) |
| 48 - | × (heavy) |

Each item except detachability was given the following points. The points were totalled and taken as the total evaluation score of a formed capsule.

**Table 7**

| item | evaluation score |
|---|---|
| ⊙⊙ | 5 |
| ⊙○ | 4 |
| ⊙ | 3 |
| ○ | 2 |
| Δ | 1 |
| × | 0 |

### [Starting materials used]

The materials to be used in Examples and Comparative Examples are explained in the following.
(A) hydroxypropyletherified starch (NIPPON STARCH CHEMICAL CO., LTD., G-800, water content 13%)
(B) (-polyglutamic acid (Ajinomoto Co., Inc., food additive, molecular weight 28,000, water content 5%),
(C) Water includes water content of (A) and (B).
(D) glycerol (Lion Corporation, food additive, water content 0%)
(E) a glycerin fatty acid ester (Riken Vitamin Co., Ltd., G-002, water content 0%)
(F) palm oil (J-OIL MILLS. INC., product number Fry UP 201, water content 0%)

In Examples and Comparative Examples, the amount converted to a solid content is shown, and water is a total of the amount used for dissolving each solid content and added water.

### [Examples 1-4]

In Examples 1-4, adhesiveness, film strength, elongation and transparency at various ratios of (A) and (B) components were evaluated by the above-mentioned measurement methods. The results are as shown in Table 8.

**Table 8**

| | Ex. 1 | Ex. 2 | Ex. 3 | Ex. 4 |
|---|---|---|---|---|
| (A) | 54% | 51% | 48% | 42% |
| (B) | 6% | 9% | 12% | 18% |
| (C) | 40% | 40% | 40% | 40% |
| total | 100% | 100% | 100% | 100% |
| (A) / (A) + (B) | 90% | 85% | 80% | 70% |
| (B) / (A) + (B) | 10% | 15% | 20% | 30% |
| adhesiveness | ○ | ⊙ | ⊙ | ○ |
| transparency | ○ | ○ | ○ | ○ |
| strength | ⊙ | ⊙ | ○ | Δ |
| elongation | ○ | ○ | ○ | ○ |
| burden on drying step | ⊙ | ⊙ | ⊙ | ⊙ |
| total evaluation | 12 | 13 | 12 | 10 |

### [Examples 5-8 and Comparative Examples 1-2]

In Examples 5-8, adhesiveness, film strength, elongation and transparency were evaluated at various ratios of (A) and (B) components and with addition of component (D), according to the above-mentioned measurement method. In Comparative Examples 1-2, adhesiveness, film strength, elongation and transparency were evaluated without component (A) or (B), according to the above-mentioned measurement method. The results are as shown in Table 9.

**Table 9**

| | Ex. 5 | Ex. 6 | Ex. 7 | Ex. 8 | Com. Ex. 1 | Com. Ex. 2 |
|---|---|---|---|---|---|---|
| (A) | 46% | 42% | 37% | 32% | 51% | 0% |
| (B) | 5% | 10% | 15% | 20% | 0% | 55% |
| (C) | 41% | 41% | 40% | 40% | 42% | 37% |
| (D) | 8% | 8% | 8% | 8% | 8% | 8% |
| total | 100% | 100% | 100% | 100% | 100% | 100% |
| (A)/(A)+(B) | 90.2% | 80.8% | 71.2% | 61.5% | 100.0% | 0.0% |
| (B)/(A)+(B) | 9.8% | 19.2% | 28.8% | 38.5% | 0.0% | 100.0% |
| adhesiveness | ○ | ⊙ | ○ | ○ | × | - |
| transparency | ○ | ⊙ | ○ | ⊙ | × | - |
| strength | ⊙ | ⊙ | ⊙ | Δ | ○ | - |
| elongation | ○ | ⊙ | ○ | ⊙ | ○ | - |
| burden on drying step | ⊙ | ⊙ | ⊙ | ⊙ | ○ | ⊙ |
| total evaluation | 12 | 15 | 12 | 12 | 6 | 2 |

| | | | | | | |
|---|---|---|---|---|---|---|
| - means measurement was not possible due to liquid. | | | | | | |

### [Examples 9-14 and Comparative Examples 3-6]

In Examples 9-14, capsule shell sheets were prepared in the same manner as in Examples 1-4 and at various amount of water added from 33% to 47%, and evaluated for the adhesiveness, film strength, elongation and transparency. In Comparative Examples 3-6, capsule shell sheets were prepared in the same manner as in Examples 1-4 using a composition free of PGA and evaluated for the adhesiveness, film strength, elongation and transparency. The results are as shown in Table 10.

**Table 10-1**

| | Ex. 9 | Ex. 10 | Ex. 11 | Ex. 12 | Ex. 13 | Ex. 14 |
|---|---|---|---|---|---|---|
| (A) | 48% | 46% | 43% | 42% | 40% | 37% |
| (B) | 11% | 11% | 11% | 10% | 10% | 9% |
| (C) | 33% | 35% | 38% | 41% | 43% | 47% |
| (D) | 8% | 8% | 8% | 7% | 7% | 7% |
| total | 100% | 100% | 100% | 100% | 100% | 100% |
| (A) / (A) + (B) | 81.3% | 80.7% | 79.6% | 80.8% | 80.0% | 80.4% |
| (B)/(A)+(B) | 18.6% | 19.3% | 20.4% | 19.2% | 20.0% | 19.6% |
| adhesiveness | Δ | ○ | ○ | ⊙ | ○ | ○ |
| transparency | ○ | ○ | ⊙ | ⊙ | ⊙ | ⊙ |
| strength | ⊙ | ⊙ | ⊙ | ⊙ | ○ | Δ |
| elongation | ○ | ○ | ⊙ | ⊙ | ⊙ | ⊙ |
| burden on drying step | ⊙○ | ⊙○ | ⊙ | ⊙ | ⊙ | ○ |
| total evaluation | 12 | 13 | 14 | 15 | 12 | 11 |

**Table 10-2**

| Comparative Example 3 | Comparative Example 4 | Comparative Example 5 | Comparative Example 6 |
|---|---|---|---|
| 51% | 49% | 45% | 44% |
| 0% | 0% | 0% | 0% |
| 42% | 44% | 48% | 50% |
| 7% | 7% | 7% | 6% |
| 100% | 100% | 100% | 100% |
| 100% | 100% | 100% | 100% |
| 0% | 0% | 0% | 0% |
| - | Δ | ○ | Δ |
| - | × | Δ | ○ |
| - | ○ | Δ | Δ |
| - | × | Δ | ○ |
| ○ | ○ | × | × |
| 2 | 5 | 5 | 6 |

| | | | |
|---|---|---|---|
| - means sheet could not be produced because solid content was not dissolved in water. | | | |

### [Examples 15-21]

In Examples 15-21, component (D) was increased, component (C) was reduced, and adhesiveness, film strength, elongation and transparency were evaluated by the above-mentioned measurement methods. The results are as shown in Table 11.

**Table 11**

| | Ex. 15 | Ex. 16 | Ex. 17 | Ex. 18 | Ex. 19 | Ex. 20 | Ex. 21 |
|---|---|---|---|---|---|---|---|
| (A) | 45% | 44% | 42% | 39% | 36% | 39% | 40% |
| (B) | 11% | 10% | 10% | 9% | 9% | 10% | 10% |
| (C) | 41% | 41% | 41% | 40% | 40% | 34% | 32% |
| (D) | 4% | 5% | 8% | 12% | 15% | 17% | 18% |
| total | 100% | 100% | 100% | 100% | 100% | 100% | 100% |
| (B)/(A)+(B) | 19.6% | 18.5% | 19.2% | 18.8% | 20.0% | 19.4% | 20.0% |
| (D)/(A)+(B) | 7.1% | 9.3% | 15.4% | 25.0% | 33.3% | 34.1% | 36.0% |
| adhesiveness | ○ | ⊙ | ⊙ | ⊙ | ○ | ⊙ | ⊙ |
| transparency | Δ | ○ | ⊙ | ⊙ | ⊙ | ⊙ | ⊙ |
| strength | ⊙ | ⊙ | ⊙ | ○ | Δ | ⊙ | ○ |
| elongation | ○ | ○ | ⊙ | ⊙ | ⊙ | ⊙ | ⊙ |
| burden on drying step | ⊙ | ⊙ | ⊙ | ⊙ | ⊙ | ⊙○ | ⊙○ |
| total evaluation | 11 | 13 | 15 | 14 | 12 | 16 | 15 |

### [Examples 22-26]

In Examples 22 - 26, capsule shell sheets were prepared in the same manner as in Examples 1-4 with various amounts of (E) glycerin fatty acid ester, and adhesiveness, film strength, elongation and transparency were evaluated. The results are as shown in Table 12.

**Table 12**

| | Ex. 22 | Ex. 23 | Ex. 24 | Ex. 2.5 | Ex. 26 |
|---|---|---|---|---|---|
| (A) | 41% | 40% | 37% | 36% | 35% |
| (B) | 10% | 10% | 9% | 9% | 8% |
| (C) | 41% | 41% | 41% | 41% | 41% |
| (D) | 8% | 7% | 7% | 7% | 6% |
| (E) | 1% | 3% | 6% | 8% | 10% |
| total | 100% | 100% | 100% | 100% | 100% |
| (B)/(A)+(B) | 19.6% | 20.0% | 19.6% | 20.0% | 18.6% |
| (D)/(A)+(B) | 15.7% | 14.0% | 15.2% | 15.6% | 14.0% |
| (E)/(A)+(B) | 2.0% | 6.0% | 13.0% | 17.8% | 23.3% |
| adhesiveness | ⊙ | ⊙ | ⊙ | ⊙ | ⊙ |
| transparency | ⊙ | ⊙ | ⊙ | ⊙ | ⊙ |
| strength | ⊙ | ○⊙ | ⊙⊙ | ⊙ | ○ |
| elongation | ⊙ | ⊙ | ○⊙ | ○⊙ | ○⊙ |
| burden on drying step | ⊙ | ⊙ | ⊙ | ⊙ | ⊙ |
| total evaluation | 15 | 16 | 18 | 16 | 15 |

### [Examples 27-30]

In Examples 27 - 30, the evaluation was performed with various ratios of oil. Oil was added and the property was measured. The results are as shown in Table 13.

**Table 13**

| | Ex. 27 | Ex. 28 | Ex. 29 | Ex. 30 |
|---|---|---|---|---|
| (A) | 37% | 37% | 37% | 36% |
| (B) | 9% | 9% | 9% | 9% |
| (C) | 41% | 41% | 42% | 43% |
| (D) | 7% | 7% | 7% | 7% |
| (E) | 6% | 6% | 6% | 6% |
| (F) | 0.25% | 0.50% | 1.0% | 2.0% |
| total | 100% | 100% | 100% | 100% |
| (B)/(A)+(B) | 19.6% | 19.6% | 19.6% | 20.0% |
| (D) / (A)+(B) | 15.2% | 15.2% | 15.2% | 15.6% |
| (E) / (A)+(B) | 13.0% | 13.0% | 13.0% | 13.3% |
| (F)/(A)+(B) | 0.5% | 1.1% | 2.2% | 4.4% |
| adhesiveness | ⊙ | ⊙ | ⊙ | ⊙ |
| transparency | ⊙ | ⊙ | ⊙ | ⊙ |
| strength | ○⊙ | ⊙⊙ | ○ | Δ |
| elongation | ○⊙ | ⊙⊙ | ⊙ | ○ |
| burden on drying step | ⊙ | ⊙ | ○ | ○ |
| total evaluation | 17 | 19 | 13 | 11 |
| detachability | ⊙ | ⊙⊙ | ⊙⊙ | ⊙⊙ |

Production Examples performed in the state close to the actual production are shown below. The evaluation methods are first explained.

### <Elongation and elastic modulus>

A test piece (6 mmx60 mm) was prepared from a sheet obtained from a conical die. The test piece was set to TENSILON (ORIENTEC, RTC-1250A) and elongation (ratio of elongated part relative to original sheet) and elastic modulus (MPa) were measured at a tensile rate of 50 mm/min.

In addition, the elongation and elastic modulus of a sheet suitable for capsulation were defined in three levels as follows.

### 1. Elongation

O...not less than 80%
Δ...79 - 40%
×... not more than 39%
2. Elastic modulus
○... not more than 0.39 MPa
Δ...0.40 - 0.79 MPa
×... not less than 0.80 MPa

### <Adhesiveness>

Two pieces of sheets were applied to a capsule forming machine. When the sheets were adhered to each other, the bonded surface was free of concaves and convexes, and the content filled therein did not leak out after contact with a hand, the capsule was evaluated to "have adhesiveness". When the content could be filled in but the bonded surface had concaves and convexes, and the content leaked out after contact with a hand, the capsule was evaluated to have "no adhesiveness".

### <Stamping>

To produce a capsule by stamping, a capsule shell needs to have sufficient strength and detachability from a mold. When a capsule could be formed by staming two pieces of sheets using the mold of a capsule forming machine, the sheet was evaluated as "good" and a sheet failed to provide a capsule by stamping (undetachable from the sheet) was evaluated as "no good".

### <State after drying>

The hardness and elasticity of a capsule after drying (water content of capsule shell 10 - 12%) were evaluated by the feel of hand.

### <Transparency>

The transparency of capsule was visually evaluated into "transparent" and "semitransparent".

### <Stickiness of capsule after forming>

The stickiness was evaluated in 3 levels shown in the following Table 14 by the feel of hand.

**Table 14**

| | |
|---|---|
| no stickiness at all | ⊙ |
| almost no stickiness | ○ |
| Sticky | × |

### [Production Example 1]

The starting materials mixed according to the following formulation were applied to an extruder (real machine) and treated at a high pressure. Using a conical die set at the extruder outlet, a 1 mm thick sheet was prepared.

The operation conditions of an extruder (2 axis extruder EA-20 manufactured by Suehiro EPM Corporation) were rotation: 80 rpm, pressure: 2.0 kg/cm², carrier part temperature: 90°C, temperature of temperature rise part: 110°C. However, only in Production Example 1, Production Comparative Examples 1 and 2, the real amounts used of the shell materials fed into the extruder are indicated. In (), a breakdown of the net solid amount and the amount of water is indicated. As for the relationship in amount of each component of a composition for a capsule shell, calculated values are indicated hereunder.

| | | |
|---|---|---|
| water added | | 30.8 wt% |
| hydroxypropyletherified starch (G-800, manufactured by NIPPON STARCH CHEMICAL CO., LTD.): | | 46.2 wt% |
| | (net solid amount 40.2 wt%+water 6.0 wt%) | |
| hydroxypropyletherified starch decomposed product (PKW, manufactured by NIPPON STARCH CHEMICAL CO., LTD.): | | 7.4 wt% |
| | (net solid amount 6.4 wt%+water 1.0 wt%) | |
| γ-polyglutamic acid (food additive, manufactured by Ajinomoto Co., Inc., molecular weight 28,000): | | 7.4 wt% |
| | (net solid amount 7.0 wt%+water 0.4 wt%) | |
| glycerol (food additive, manufactured by Lion Corporation): | | 7.4 wt% |
| propylene glycol (food additive, manufactured by JUNSEI CHEMICAL CO., LTD.): | | 0.3 wt% |
| glycerin fatty acid ester (G-002, manufactured by Riken | | |
| Vitamin Co., Ltd.): | 0.5 wt% | |
| (A)/(A)+(B) | 86.9% | |
| (B)/(A)+(B) | 13.1% | |
| total amount of component (C) | 38.1 wt% | |
| (D)/(A)+(B) | 13.8% | |
| (E)/(A)+(B) | 0.9% | |

### [Production Example 2]

A sheet was prepared in the same manner as in Production Example 1 except that the material composition alone was changed.

| | | |
|---|---|---|
| Weight composition of shell materials fed into the extruder water added | | 23.1 wt% |
| hydroxypropyletherified starch (G-800, manufactured by NIPPON STARCH CHEMICAL CO., LTD.): | | 53.8 wt% |
| | (net solid amount 46.8 wt%+water 7.0 wt%) | |
| K-carageenan (WR-80-J, manufactured by Sansho Co., Ltd.): | | 0.8 wt% |
| | (net solid amount 0.7 wt%+water 0.1 wt%) | |
| γ-polyglutamic acid (food additive, manufactured by Ajinomoto Co., Inc., molecular weight 28,000): | | 7.7 wt% |
| | (net solid amount 7.3 wt%+water 0.4 wt%) | |
| glycerol (food additive, manufactured by Lion Corporation): | | 13.8 wt% |
| glycerin fatty acid ester (G-002, manufactured by Riken Vitamin Co., Ltd.): | | 0.8 wt% |
| (A)/(A)+(B) | 86.7% | |
| (B) / (A) + (B) | 13.3% | |
| total amount of component (C) | 30.6 wt% | |
| (D)/(A)+(B) | 25.2% | |
| (E)/(A)+(B) | 1.5% | |
| ((x-carageenan)/(A) 1.5%) | | |

### [Production Comparative Example 1]

A sheet was prepared in the same manner as in Production Example 1 except that the material composition alone was changed. The motor load was 10A.
Weight composition of shell materials fed into the extruder (amount of water does not include the amount of water contained in each material).

| | | |
|---|---|---|
| water added | | 30.8 wt% |
| hydroxypropyletherified starch (G-800, manufactured by NIPPON STARCH CHEMICAL CO., LTD.): | | 59.9 wt% |
| | (net solid amount 52.1 wt%+water 7.8 wt%) | |
| hydroxypropyletherified starch decomposed product (PKW, manufactured by NIPPON STARCH CHEMICAL CO., LTD.): | | 3.0 wt% |
| | (net solid amount 2.6 wt%+water 0.4 wt%) | |
| glycerol (food additive, manufactured by Lion Corporation): | | 6.0 wt% |
| propylene glycol (food additive, manufactured by JUNSEI CHEMICAL CO., LTD.): | | 0.3 wt.% |
| (A)/(A)+(B) | 100.0% | |
| (B)/(A)+(B) | 0.0% | |
| total amount of component (C) | 39.0 wt% | |
| (D)/(A)+(B) | 11.0% | |
| (E)/(A)+(B) | 0.5% | |

### [Production Comparative Example 2]

A sheet was prepared in the same manner as in Production Example 1 except that the material composition alone was changed. The motor load was 5.2A. The amount of water including the amount of water contained in the powder was 40% relative to the total amount.
Weight composition of shell materials fed into the extruder

| | | |
|---|---|---|
| water added | | 30.8 wt% |
| hydroxypropyletherified starch (G-800, manufactured by NIPPON STARCH CHEMICAL CO., LTD.): | | 49.9 wt% |
| | (net solid amount 43.4 wt%+water 6.5 wt%) | |
| hydroxypropyletherified starch decomposed product (PKW, manufactured by NIPPON STARCH CHEMICAL CO., LTD.): | | 11.1 wt% |
| | (net solid amount 9.7 wt%+water 1.4 wt%) | |
| glycerol (food additive, manufactured by Lion Corporation): | | 7.4 wt% |

| | | |
|---|---|---|
| propylene glycol (food additive, manufactured by JUNSEI CHEMICAL CO., LTD.): | | 0.3 wt% |
| glycerin fatty acid ester (G-002, manufactured by Riken Vitamin Co., Ltd.): | | 0.5 wt% |
| (A)/(A)+(B) | 100.0% | |
| (B)/(A)+(B) | 0.0% | |
| total amount of component (C) | 38.7 wt% | |
| (D)/(A)+(B) | 13.9% | |
| (E)/(A)+(B) | 0.9% | |

As Production Example 1 (containing polyglutamic acid), Production Example 2 (containing polyglutamic acid, κ-carageenan), Production Comparative Examples 1, 2 (without polyglutamic acid), soft capsules were prepared by a continuous rotary die capsule filling machine using shell sheets prepared as mentioned above and a capsule content (medium chain fatty acid), and evaluated. The results are shown in Table 15.

**Table 15**

| Property evaluation of capsule shell composition | | | | |
|---|---|---|---|---|
| | Production Example 1 | Production Example 2 | Production Comparative Example 1 | Production Comparative Example 2 |
| elongation (%) | ○ (81) | ○ (95) | × (38) | Δ (62) |
| elastic modulus (MPa) | ○ (0.33) | ○ (0.27) | × (1.80) | × (1.90) |
| transparency | transparent | transparent | semitransparent | semitransparent |
| adhesiveness | yes | yes | none | none |
| stamping (strength, detachability) | good | good | no good | no good |
| stickiness of capsule after forming | ○ | ⊙ | ○ | ○ |
| state after drying | soft | soft | stiff | stiff |

As shown in Table 15, without addition of polyglutamic acid, elongation and elastic modulus become insufficient, the transparency, adhesiveness and stamping performance become poor, and only a capsule stiff after drying can be obtained (Production Comparative Examples 1, 2). However, it has been clarified that, by the addition of polyglutamic acid, the elongation of a sheet can be improved, the elastic modulus becomes small, a sheet more suitable for capsule forming can be produced, and the addition is effective for adhesiveness between sheets made of the shell composition, as well as the softness of capsule after drying (Production Example 1). Moreover, it has also been clarified that the elongation of a shell composition can be improved, a capsule can be produced by stamping using a mold of a capsule forming machine, and the transparency can also be improved. In addition, by the addition of κ-carageenan by 1.5% as component A, the stickiness immediately after forming can be prevented (Production Example 2)

### Industrial Applicability

While a composition for a capsule shell generally contains water as an essential component, the composition for a capsule shell of the present invention can reduce the water content of the composition for a capsule shell by a combined use of vegetable polysaccharides and polyglutamic acid. Therefore, the composition is advantageous in that the drying time can be shortened during formation of a shell sheet for the preparation of capsules, or during production of capsules, and the like. Since the shell sheet obtained from the composition is rich in flexibility and superior in tensile strength, capsules produced by a forming machine are not deformed, and capsules having a rugby ball shape like a gelatin capsule can be produced. Moreover, since capsule shell sheets show superior adhesiveness to each other, a suspension difficult to be filled in conventional vegetable capsules can be encapsulated. Shell sheets and capsule preparations obtained by processing the composition for a capsule shell of the present invention are entirely made of edible components and highly safe, and can be applied to various uses as biocompatible materials.

The capsule preparation of the present invention is widely useful in the fields of pharmaceutical product, food, health food and the like, as a practical plant-derived capsule replacing the existing gelatin capsules.

## Claims

1. A composition for producing a soft capsule shell, which comprises (A) a vegetable polysaccharide and (B) polyglutamic acid.

2. The composition of claim 1, which further comprises (C) water.

3. The composition of claim 1 or 2, which further comprises (D) a plasticizer.

4. The composition of any of claims 1 to 3, which further comprises (E) a glycerin fatty acid ester.

5. The composition of any of claims 1 to 4, which further comprises (F) an oil.

6. The composition of any of claims 1 to 5, wherein (A) is a starch.

7. The composition of claim 6, wherein the starch is selected from tapioca starch, corn starch, waxy corn starch, rice starch and wheat starch.

8. The composition of any of claims 1 to 7, wherein (A) is a modified vegetable polysaccharide.

9. The composition according to any one of the preceding claims wherein the mixing ratio (mass ratio) of (A) and (B) is 95:5 - 60:40.

10. A method of producing a soft capsule shell sheet comprising processing the composition of any one of the preceding claims with an extruder.

11. A method of producing a soft capsule shell comprising producing a soft capsule shell sheet by the method of claim 10 and forming the sheet into a capsule shell.

12. The method of claim 11 further comprising including contents components in the soft capsule shell to form a soft capsule preparation.

13. A soft capsule shell sheet, a soft capsule shell, or a soft capsule preparation as obtainable by the method of claim 10, claim 11 or claim 12 respectively.

14. A soft capsule shell sheet, a soft capsule shell, or a soft capsule preparation comprising the composition for preparing a capsule shell according to any one of claims 1 to 9.

## Patentansprüche

1. Zusammensetzung zum Herstellen einer Weichkapselschale, welche (A) ein pflanzliches Polysaccharid und (B) Polyglutaminsäure enthält.

2. Zusammensetzung nach Anspruch 1, die außerdem (C) Wasser enthält.

3. Zusammensetzung nach Anspruch 1 oder 2, die außerdem (D) einen Weichmacher enthält.

4. Zusammensetzung nach einem der Ansprüche 1 bis 3, die außerdem (E) einen Glycerinfettsäureester enthält.

5. Zusammensetzung nach einem der Ansprüche 1 bis 4, die außerdem (F) ein Öl enthält.

6. Zusammensetzung nach einem der Ansprüche 1 bis 5, wobei (A) eine Stärke ist.

7. Zusammensetzung nach Anspruch 6, wobei die Stärke unter Tapiocastärke, Maisstärke, Wachsmaisstärke, Reisstärke und Weizenstärke ausgewählt ist.

8. Zusammensetzung nach einem der Ansprüche 1 bis 7, wobei (A) ein modifiziertes pflanzliches Polysaccharid ist.

9. Zusammensetzung nach einem der vorstehenden Ansprüche, wobei das Mischungsverhältnis (Massenverhältnis) von (A) und (B) 95:5 bis 60:40 ist.

10. Verfahren zum Herstellen einer Weichkapselschalenplatte, welches das Verarbeiten der Zusammensetzung nach einem der vorstehenden Ansprüche mit einem Extruder umfasst.

11. Verfahren zum Herstellen einer Weichkapselschale, welches das Herstellen einer Weichkapselschalenplatte durch das Verfahren nach Anspruch 10 und das Formen der Platte in eine Kapselschale umfasst.

12. Verfahren nach Anspruch 11, das außerdem das Einverleiben von Zusammensetzungsbestandteilen in die Weichkapselschale unter Bildung einer Weichkapselzubereitung umfasst.

13. Weichkapselschalenplatte, Weichkapselschale oder Weichkapselschalenzubereitung, erhältlich durch das Verfahren nach Anspruch 10, Anspruch 11 bzw. Anspruch 12.

14. Weichkapselschalenplatte, Weichkapselschale oder Weichkapselzubereitung, welche die Zusammensetzung für das Herstellen einer Kapselschale nach einem der Ansprüche 1 bis 9 umfasst.

## Revendications

1. Composition pour la production d'une coque de capsule souple qui comprend (A) un polysaccharide végétal et (B) un acide polyglutamique.

2. Composition selon la revendication 1, qui comprend en outre (C) de l'eau.

3. Composition selon la revendication 1 ou 2, qui comprend en outre (D) un plastifiant.

4. Composition selon l'une quelconque des revendications 1 à 3, qui comprend en outre (E) un ester d'acide gras de glycérine.

5. Composition selon l'une quelconque des revendications 1 à 4, qui comprend en outre (F) une huile.

6. Composition selon l'une quelconque des revendications 1 à 5, dans laquelle (A) est un amidon.

7. Composition selon la revendication 6, dans laquelle l'amidon est choisi parmi l'amidon de tapioca, l'amidon de maïs, l'amidon de maïs cireux, l'amidon de riz et l'amidon de blé.

8. Composition selon l'une quelconque des revendications 1 à 7, dans laquelle (A) est un polysaccharide végétal modifié.

9. Composition selon l'une quelconque des revendications précédentes, dans laquelle le rapport de mélange (rapport massique) de (A) et de (B) est de 95:5 à 60:40.

10. Procédé de production d'une feuille de coque de capsule souple comprenant le traitement de la composition selon l'une quelconque des revendications précédentes avec une extrudeuse.

11. Procédé de production d'une coque de capsule souple comprenant la production d'une feuille de coque de capsule souple par le procédé selon la revendication 10 et la mise en forme de la feuille en une coque de capsule.

12. Procédé selon la revendication 11, comprenant en outre l'inclusion de composants de contenu dans la coque de capsule souple pour former une préparation de capsule souple.

13. Feuille de coque de capsule souple, coque de capsule souple ou préparation de capsule souple susceptible d'être obtenue par le procédé selon la revendication 10, la revendication 11 ou la revendication 12, respectivement.

14. Feuille de coque de capsule souple, coque de capsule souple ou préparation de capsule souple comprenant la composition pour la préparation d'une coque de capsule selon l'une quelconque des revendications 1 à 9.
